# EUROPEAN PATENT SPECIFICATION

(11) **EP 1 297 114 B1**
(45) Date of publication and mention of the grant of the patent: **28.05.2008**
(21) Application number: 01945124.4
(22) Date of filing: 11.05.2001
(51) Int. Cl.: C12N 9/12, C07K 14/435, C12N 15/85, C12N 15/12, C12N 15/64, C07K 16/18, A61K 38/16, G01N 33/68

(54) **NUCLEIC ACID MOLECULES ENCODING A PROTEIN INTERACTING WITH Ser/Thr KINASE Akt**
NUKLEINSÄURE MOLEKÜLE KODIEREND FÜR EIN PROTEIN, WELCHES MIT EINER SER/THR AKT KINASE INTERAGIERT
MOLECULES D'ACIDE NUCLEIQUE CODANT POUR UNE PROTEINE INTERAGISSANT AVEC LA SER/THR KINASE AKT

(30) Priority: 30.05.2000 EP 00111006
(43) Date of publication of application: 02.04.2003
(73) Proprietor: Mölling, Karin, Inst. für med. Virologie der Uni. Zürich, 8208 Zürich (CH)
(72) Inventor: MÖLLING, Karin, c/o Universität Zürich, 8208 Zürich (CH); ZIMMERMANN, Sven, 8044 Zürich (CH)
(74) Representative: Grünecker, Kinkeldey, Stockmair & Schwanhäusser Anwaltssozietät
(86) International application number: PCT/EP2001/005429
(87) International publication number: WO 2001/092499

(56) References cited:
- WO-A-01/05970
- WO-A-96/19579
- WO-A-97/18303
- DATABASE SWISS PROT DATABASE [Online] Accession number Q18964, SWALL:YLN2_CAEEL, 15 July 1998 (1998-07-15) "HYPOTHETICAL 46.2 KDA TRP-ASP REPEATS CONTAINING PROTEIN D2013.2 IN CHROMOSOME II, Caenorhabditis elegans." XP002180198 cited in the application
- NAGASE: "Prediction of the coding sequences of unidentified human genes" DNA RES. , vol. 7, no. 1, 2000, pages 65-73, XP000949814 cited in the application & DATABASE NCBI (BAA92673) NAGASE: 'KIAA1435 Protein'
- KONISHI H ET AL: "MOLECULAR CLONING AND CHARACTERIZATION OF A NEW MEMBER OF THE RAC PROTEIN KINASE FAMILY: ASSOCIATION OF THE PLECKSTRIN HOMOLOGY DOMAIN OF THREE TYPES OF RAC PROTEIN KINASE WITH PROTEIN KINASE C SUBSPECIES AND BETAGAMMASUBUNITS OF G PROTEINS" BIOCHEMICAL AND BIOPHYSICAL RESEARCH COMMUNICATIONS, ACADEMIC PRESS INC. ORLANDO, FL, US, vol. 216, no. 2, 13 November 1995 (1995-11-13), pages 526-534, XP002030406 ISSN: 0006-291X
- WANG QINGHUA ET AL: "Protein kinase B/Akt participates in GLUT4 translocation by insulin in L6 myoblasts" MOLECULAR AND CELLULAR BIOLOGY, AMERICAN SOCIETY FOR MICROBIOLOGY, WASHINGTON, US, vol. 19, no. 6, June 1999 (1999-06), pages 4008-4018, XP002147888 ISSN: 0270-7306

## Description

The present invention relates to nucleic acid molecules encoding a protein interacting with the Ser/Thr kinase Akt. The invention also relates to the encoded protein which is called AIP (Akt interacting protein). The invention furthermore relates to expression vectors, host cells, and an antibody for AIP as well as to pharmaceutical compositions comprising the described nucleic acid molecule, the protein, the antibody or antagonists and to methods for treating disorders associated with impaired endosomal transport.

Akt-1 (also known as PKB alpha or Rac-PK alpha) belongs to the Akt/PKB family of serine/threonine kinases and has been shown to be involved in many diverse signaling pathways (Alessi and Cohen, Curr. Opin. Genet. Dev. 8 (1998), 55-62). Akt-1 consists of an N-terminal lipid-binding pleckstrin-homology domain and a C-terminal catalytic domain. In resting cells, all Akt isoforms reside in the cytoplasm but translocate to the plasma membrane following stimulation with external ligands. Translocation and subsequent activation is induced by several different ligands including PDGF, IGF, EGF, bFGF and insulin. This activation depends on PI3 kinase activity and requires hierarchial phosphorylation of Thr308 and Ser473 of Akt-1 by PDK-1 and PDK-2, respectively (Alessi et al., Curr. Biol. 8 (1998), 69-81). Once activated, Akt-1 mediates several different functions, including prevention of apoptosis, induction of differentiation and/or proliferation, protein synthesis and the metabolic effects of insulin.
Insulin controls blood glucose levels by inducing cellular glucose uptake of fat and muscle cells. The transport of glucose across the cell surface membrane is mediated by several isoforms of a glucose transporter family among them the GLUT4 transporter (Elmendorf and Pessin, Exp. Cell Res. 253 (1999), 55-62). This is the major isoform of adipocytes and myocytes. Both of these cell types are highly responsive to insulin and harbor intracellular GLUT4-containing vesicles. Upon insulin stimulation these GLUT4 containing vesicles translocate and fuse with the plasma membrane in a process called exocytosis. The vesicle movement through the cytoplasm is believed to be abnormal in both insulin resistant and non-insulin dependent diabetis mellitus (NIDDM) (Kahn, Nature 373 (1995), 384-385) both belonging to type 11 diabetes, in which the body responds less and less well to a given amount of insulin despite of the fact that there is enough circulating insulin. The normal molecular mechanism of insulin-stimulated translocation of GLUT4 containing vesicles to the plasma membrane and the concomitant increase in glucose uptake by the affected cells are still largely unknown.
Previously, it has been shown that translocation of the GLUT4 receptor requires the activity of P13 kinase in several different experimental model systems such as rat adipocytes (Okada et al., J. Biol. Chem. 269 (1994), 3568-3573; Quon et al., J. Biol. Chem. 269 (1994), 27920-27924), 3T3-L1 adipocytes (Cheatham et al., Mol. Cell. Biol. 14 (1994); 4902-4911), L6 muscle cells (Tsakiridis et al., Endocrinology 136 (1995), 4315-4322) and rat skeletal muscle (Yeh et al., J. Biol. Chem. 270 (1995), 2107-2111). Recent reports have suggested that activation of Akt can mediate the stimulation of glucose transport by insulin. Thus, insulin-independent glucose uptake was observed in rat and murine adipocytes overexpressing either constitutive or conditionally activated Akt-1-mutants (Kohn et al., J. Biol. Chem. 273 (1998), 11937-11943; Tanti et al., Endocrinology 138 (1997), 2005-2010). Additionally, insulin treatment induced the translocation of Akt-2 to GLUT4 containing vesicles leading to phosphorylation of vesicle associated proteins (Kupiryanova and Kandror, J. Biol. Chem. 274 (1999), 1458-1464). The exact mechanism how activation and a putative translocation of Akt can lead to the movement of GLUT4 containing vesicles to the plasma membrane is, however, presently unknown. Knowledge of this mechanism and of factors involved in it would be useful to develop means for treating disorders with impaired endosomal transport, in particular diabetes or obesity.

Thus, the technical problem underlying the present invention is to identify factors involved in the Akt induced movement of GLUT4 containing vesicles to the plasma membrane and to provide nucleic acid molecules encoding such factors.

This technical problem is solved by the provision of the embodiments as characterized in the claims.

Accordingly, the present invention relates to nucleic acid molecules encoding an Akt interacting protein (AIP) selected from the group consisting of
(a) nucleic acid molecules encoding a protein which comprises the amino acid sequence indicated in SEQ ID NO: 2 or the amino acid sequence as encoded by the cDNA insert contained in plasmid DSM13510;
(b) nucleic acid molecules comprising the nucleotide sequence of the coding region indicated in SEQ ID NO: 1 or the nucleotide sequence of the coding region of the cDNA insert contained in plasmid DSM13510;
(c) nucleic acid molecules encoding a protein, the amino acid sequence of which has a homology of at least 65% to the amino acid sequence indicated in SEQ ID NO: 2;
(d) nucleic acid molecules the complementary strand of which hybridizes to a nucleic acid molecule as defined in (a) or (b) and the sequence of which has a homology of at least 70% to the coding region of the sequence shown in SEQ ID NO:1; and
(e) nucleic acid molecules, the nucleotide sequence of which deviates because of the degeneracy of the genetic code from the sequence of the nucleic acid molecules as defined in any one of (a), (b), (c) or (d).

Consequently, the present invention relates to nucleic acid molecules encoding a protein interacting with Ser/Thr kinase Akt, said molecules preferably encoding a protein comprising the amino acid sequence indicated in SEQ ID NO: 2.

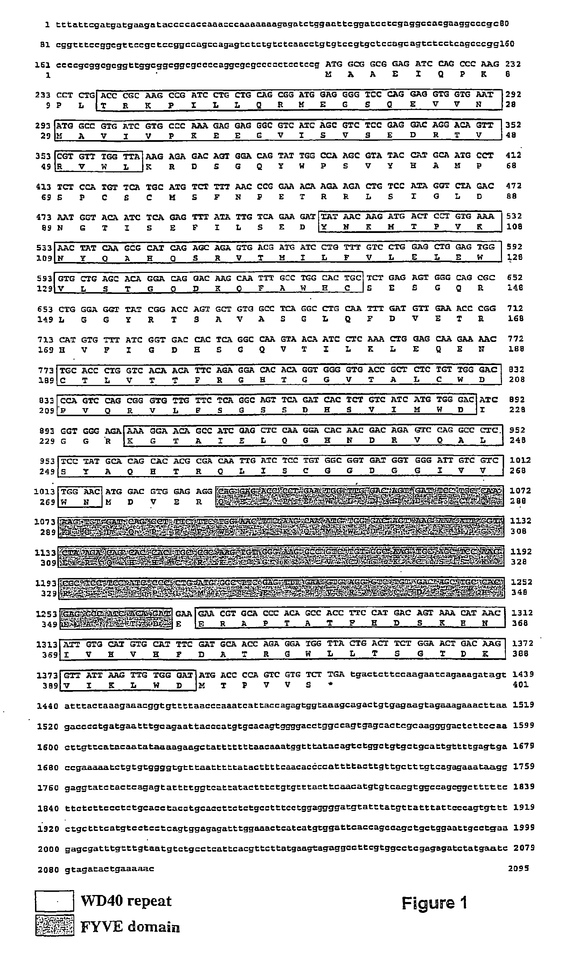

The present invention is based on the isolation of a nucleic acid molecule encoding a protein that interacts with Ser/Thr kinase Akt and which is called AIP (Akt interacting protein). The nucleotide sequence is shown in SEQ ID NO: 1 and has a length of 2095 nucleotides. It encodes a protein of 401 amino acid residues. The AIP protein shown in SEQ ID NO: 2 contains one functional FYVE domain that localizes the AIP protein to early endosomes (see Fig. 1). The so called FYVE finger has eight conserved cysteines which coordinate two Zn²⁺ ions (Stenmark and Aasland, J. Cell Sci. 112 (1999), 4175-4183). This conserved domain has been recognized as a protein module specific for phosphatidylinositols (Ptdlns) phosphorylated exclusively at position D-3 of the inositol ring (Gaullier et al., Nature 394 (1998), 432-433). These 3' phosphorylated lipids are generated from phosphoinositol by the action of a class III P13 kinase (Fruman et al., Ann. Rev. Biochem. 67 (1998), 481-507). These kinases and their respective lipid products, Ptdlns(3)P, are important for vesicular traffic and transport but little is known how this is achieved and to date, FYVE domain containing polypeptides are the only proteins that recognize these particular lipids. Among these, all of the proteins whose intracellular localization has been studied localize to early endosomes. This suggests that endosomally generated Ptdlns(3)P serve to recruit FYVE proteins specifically to early endosomes. In keeping with these implications AIP is localised to early endosomal structures in human HeLa cells that overexpress an GFP-AIP fusion protein (see Example 2).
The pharmacological inactivation of the AIP FYVE domain by incubating HeLa cells with Zn²⁺ chelators (TPEN) leads to loss of endosomal localization and a concomitant diffuse cytoplasmic staining. Accordingly, the deletion of the entire FYVE domain also leads to a cytoplasmic localization of the overexpressed AIP. This localization pattern does not depend on the relatively large GFP-fusion part of the protein, since an N-terminal myc-tag produces the same staining pattern when the cells are incubated with an anti-myc antibody and an appropriate secondary antibody to visualize myc-tagged AIP (Example 2).
In addition to its FYVE domain, AIP also has five WD-40 repeats, four of them N-terminal to the FYVE finger and one of them at the very C-terminus (see Fig. 1). WD-40 proteins contain a loosely conserved repeat of approximately 40 amino acids separated by a Trp-Asp (WD) dipeptide motif (Neer et al., Nature 371 (1994), 297-300). Many of the WD-40 proteins function in signal transduction pathways either within the cytoplasm where they participate in complex formation or in the nucleus where they function as transcriptional repressors (Smith et al., Trends Biochem. Sci. 24 (1999), 181-185). To date, none of the identified WD-40 proteins has any enzymatic activity, but merely serve as interaction or docking partners.
The AIP protein has a certain degree of homology to a hypothetical reading frame of a DNA sequence of human (Protein database of the NCBI Accession number BAA92673), to a hypothetical reading frame of D. melanogaster (Protein database of the NCBI Accession number AAF52946) and to a hypothetical reading frame of a DNA sequence of C. elegans (Swiss Prot database Accession number Q18964). The function of the proteins encoded by these hypothetical reading frames is, however, completely unknown.

In the scope of the present invention the term AIP refers to a protein which is capable to interact with a Ser/Thr kinase Akt. The term "interact" in this context means that the protein is able to bind to Akt, in particular when tested in a yeast two-hybrid screening assay. This assay is described in detail in Example 1. Preferably, the AIP protein is able to bind to Akt when tested in the two-hybrid screening assay as described in Example 1.
The term "Akt" protein referred to herein is an intracellular Ser/Thr kinase Akt also known as PKB or RAC-PK. It comprises all known isoforms of Akt protein, e.g., isoform Akt-1, Akt-2 and Akt-3. An Akt protein is characterized by the following properties: All three Akt isoforms, which are so far known, are more than 80% homologous in primary structure and contain an N-terminal domain, called Akt-Homology (AH) domain whose core motif is a pleckstrin homology (PH) domain that belongs to a domain superfamily whose prototype was originally observed in pleckstrin (Datta et al., Mol. Cell Biol. 15 (1995), 2304-2310; Hemmings, Science 275 (1997), 1899; Musacchio et al., Trends Biochem. Sci. 18 (1993), 343-348). The PH domain is followed by a highly conserved catalytic serine/threonine kinase domain and a short C-terminal regulatory region. All isoforms possess conserved threonine and serine residues (T308/S473 in Akt-1; T309/S474 in Akt-2; T302/S472 in Akt-3) whose phosphorylation state is critical for Akt activation. Full activation of all Akt proteins depends on PI3-kinase that generates phospholipids that recruit Akt to the plasma membrane and activate the Akt phosphorylating kinases such as PDK1 and 2 (Alessi et al., Curr. Biol. 8 (1998), 69-81). Akt proteins are described in the literature (see, e.g., Masure et al., Eur. J. Biochem. 265 (1999), 353-360; Jones et al., Cell Regul. 2 (1991), 1001-1009; Jones et al., Proc. Natl. Acad. Sci. USA 88 (1991), 4171-4175; Coffer and Woodgett, Eur. J. Biochem. 201 (1991), 475-481; Brodbeck et al., J. Biol. Chem. 274 (1999), 9133-9136; Cheng et al., Proc. Natl. Acad. Sci. USA 89 (1992), 9267-9271; Nakatani et al., Biochem. Biophys. Res. Commun. 2577 (1999), 906-910). DNA sequences encoding the different isoforms are, e.g., disclosed for Akt-1 in Jones et al., (Proc. Natl. Acad. Sci. USA 88 (1991), 4171-4175) and in Swissprot Acc. No. P31749, for Akt-2 in Jones et al. (Cell Regul. 2 (1991), 1001-1009) and Swissprot Acc. No. P31751, and for Akt-3 in Brodbeck et al., (loc. cit.) and in Swissprot Acc. No. Q9Y243. In a preferred embodiment the term "Akt" or "Akt protein" refers to an Akt protein of the isoform 1, i.e. Akt-1. This isoform is described, e.g., in Bellacosa et al. (Science 254 (1991), 274-277) and in Staal (Proc. Natl. Acad. Sci. USA 84 (1987), 5034-5037).

The ability of the AIP protein to bind to Akt can also be tested by an assay as described in Example 3, i.e. a pull-down assay. In this assay one of the presumably interacting proteins is expressed in bacteria where it can be easily purified in larger quantities. Purification is, for example, achieved by fusing a given protein to the glutathione-S-transferase protein (GST). GST binds with high affinity to glutathione preferably immobilised to agarose beads. The incubation of these beads with bacterial lysates that contain the desired protein fused to GST leads to selective coupling and purification of the fusion protein. After a certain incubation time, unbound proteins are washed away, and the immobilised fusion protein can be used to be incubated with lysates from cells that express the interacting protein. Once again, unbound proteins are washed away, and remaining proteins are boiled in SDS-Lammli buffer, subjected to SDS-PAGE and western blotting. In this particular case, AIP was N-terminal fused to GST, purified as described above and incubated with lysates from Akt overexpressing 293 cells.

In a preferred embodiment the AIP protein, when present in cells, in particular in human cells, is located at endosomal vesicles. This localization can be determined, e.g., by an assay as described in Example 2, i.e. an in situ assay using AIP specific antibodies or antibodies specifically recognizing a tag attached to the AIP protein. Moreover, in another preferred embodiment the ability of AIP to localize to endosomal vesicles can be destroyed by the addition of an inhibitor for FYVE domains, such as Zn²⁺ chelators, e.g. TPEN. This can be determined by an assay as described, e.g., in Example 2.

In another preferred embodiment, the AIP protein according to the invention comprises one functional FYVE finger domain (see above).

In a further preferred embodiment the AIP protein contains WD-40 repeats (see above), preferably five WD-40 repeats. Most preferably, four of the WD-40 repeats are located N-terminal to the FYVE domain and one is located at the C-terminus.
Furthermore, the AIP protein preferably has a molecular weight of 40 to 50 kD when determined by SDS polyacrylamid gel electrophoresis or calculated on the basis of the amino acid sequence, more preferably of 43 to 46 kD and particularly preferred about 44 kD.

In a particularly preferred embodiment the inactivation of the AIP protein by inhibitors of FYVE domains, such as Zn²⁺ chelators, e.g. TPEN, leads to a prevention of insulin dependent glucose uptake in differentiated skeletal muscle cells. This can be tested by an assay as described, e.g., in Example 4.

The invention in particular relates to nucleic acid molecules containing the nucleotide sequence indicated under SEQ ID NO: 1 or contained in plasmid DSM13510 or a part thereof or a corresponding ribonucleotide sequence.

Moreover, the present invention relates to nucleic acid molecules which encode an AIP protein and the complementary strand of which hybridizes with one of the above-described molecules.
The present invention also relates to nucleic acid molecules which encode a protein, which has a homology, that is to say a sequence identity, of at least 50%, preferably of at least 60%, more preferably of at least 62%, even more preferably of at least 65% and particularly preferred of at least 70%, especially preferred of at least 80% and even more preferred of at least 90% to the entire amino acid sequence as indicated in SEQ ID NO: 2 or as encoded by plasmid DSM13510 the protein being an AIP protein.
Moreover, the present invention relates to nucleic acid molecules which encode an AIP protein and the nucleotide sequence of which has a homology, that is to say a sequence identity, of at least 40%, preferably of at least 50%, more preferably of at least 60%, even more preferably of more than 65%, in particular of at least 70%, especially preferred of at least 80%, in particular of at least 90% and even more preferred of at least 95% when compared to the coding region of the sequence shown in SEQ ID NO: 1 or compared to the coding region contained in the cDNA insert of plasmid DSM13510.
The present invention also relates to nucleic acid molecules, which encode an AIP protein and the sequence of which deviates from the nucleotide sequences of the above-described nucleic acid molecules due to the degeneracy of the genetic code.
The invention also relates to nucleic acid molecules comprising a nucleotide sequence which is complementary to the whole or a part of one of the above-mentioned sequences.

In the context of the present invention the term "hybridization" means hybridization under conventional hybridization conditions, preferably under stringent conditions, as for instance described in Sambrook et al., Molecular Cloning, A Laboratory Manual, 2nd edition (1989) Cold Spring Harbor Laboratory Press, Cold Spring Harbor, NY. In an especially preferred embodiment the term "hybridization" means that hybridization occurs under the following conditions:

| | |
|---|---|
| Hybridization buffer: | 2 x SSC; 10 x Denhardt solution (Fikoll 400 + PEG + BSA; ratio 1:1:1); 0.1% SDS; 5 mM EDTA; 50 mM Na₂HPO₄; 250 µg/ml of herring sperm DNA; 50 µg/ml of tRNA; or 0.25 M of sodium phosphate buffer, pH 7.2; 1 mM EDTA 7% SDS |
| Hybridization temperature T | = 60°C |
| Washing buffer: | 2 x SSC; 0.1% SDS |
| Washing temperature T | = 60°C. |

Nucleic acid molecules which hybridize with the nucleic acid molecules of the invention can, in principle, encode an AIP protein from any organism expressing such proteins or can encode modified versions thereof.
Nucleic acid molecules which hybridize with the molecules of the invention can for instance be isolated from genomic libraries or cDNA libraries of bacteria, fungi, plants or animals. Preferably, such molecules are from animal origin, particularly preferred from human orgigin. Alternatively, they can be prepared by genetic engineering or chemical synthesis.
Such nucleic acid molecules may be identified and isolated by using the molecules of the invention or parts of these molecules or reverse complements of these molecules, for instance by hybridization according to standard methods (see for instance Sambrook et al., 1989, Molecular Cloning. A Laboratory Manual, 2nd edition, Cold Spring Harbor Laboratory Press, Cold Spring Harbor, NY).
Nucleic acid molecules comprising the same or substantially the same nucleotide sequence as indicated in SEQ ID NO: 1 or parts thereof can, for instance, be used as hybridization probes. The fragments used as hybridization probes can also be synthetic fragments which are prepared by usual synthesis techniques, and the sequence of which is substantially identical with that of a nucleic acid molecule according to the invention.
The molecules hybridizing with the nucleic acid molecules of the invention also comprise fragments, derivatives and allelic variants of the above-described nucleic acid molecules encoding an AIP protein. Herein, fragments are understood to mean parts of the nucleic acid molecules which are long enough to encode the described protein, preferably showing the biological activity of an AIP protein described above, e.g. being capable to interact with Ser/Thr kinase Akt. In this context, the term derivative means that the sequences of these molecules differ from the sequences of the above-described nucleic acid molecules in one or more positions and show a high degree of homology to these sequences. In this context, homology means a sequence identity of at least 40%, in particular an identity of at least 60%, preferably of more than 65%, even more preferably of at least 70%, in particular of at least 80%, more preferably of at least 90% and particularly preferred of more than 95%. Deviations from the above-described nucleic acid molecules may have been produced, e.g., by deletion, substitution, insertion and/or recombination.
Preferably, the degree of homology is determined by comparing the respective sequence with the nucleotide sequence of the coding region of SEQ ID No: 1. When the sequences which are compared do not have the same length, the degree of homology preferably refers to the percentage of nucleotide residues in the shorter sequence which are identical to nucleotide residues in the longer sequence. The degree of homology can be determined conventionally using known computer programs such as the DNASTAR program with the Clustalw analysis. This program can be obtained from DNASTAR, Inc., 1228 South Park Street, Madison, WI 53715 or from DNASTAR, Ltd., Abacus House, West Ealing, London W13 0AS UK (support@dnastar.com) and is accessible at the server of the EMBL outstation.
When using the Clustal analysis method to determine whether a particular sequence is, for instance, 80% identical to a reference sequence the settings are preferably as follows: PAIRGAP: 0.05; MATRIX: blosum; GAP OPEN: 10; END GAPS: 10; GAP EXTENSION: 0.05; GAP DISTANCE: 0.05; KTUP: 1; WINDOW LENGTH: 0; TOPDIAG: 1.
Furthermore, homology means preferably that the encoded protein displays a sequence identity of at least 50%, more preferably of at least 60%, even more preferably of at least 62%, in particular of at least 65%, particularly preferred of at least 70%, especially preferred of at least 80% and even more preferred of at least 90% to the amino acid sequence depicted under SEQ ID NO: 2 or as encoded by plasmid DSM13510.
Preferably, sequences hybridizing to a nucleic acid molecule according to the invention comprise a region of homology of at least 90%, preferably of at least 93%, more preferably of at least 95%, still more preferably of at least 98% and particularly preferred of at least 99% identity to an above-described nucleic acid molecule, wherein this region of homology has a length of at least 500 nucleotides, more preferably of at least 750 nucleotides, even more preferably of at least 800 nucleotides, particularly preferred of at least 900 nucleotides and most preferably of at least 1000 nucleotides.
Homology, moreover, means that there is a functional and/or structural equivalence between the corresponding nucleic acid molecules or proteins encoded thereby. Nucleic acid molecules which are homologous to the above-described molecules and represent derivatives of these molecules are normally variations of these molecules which represent modifications having the same biological function. They may be either naturally occurring variations, for instance sequences from other ecotypes, varieties, species, etc., or mutations, and said mutations may have formed naturally or may have been produced by deliberate mutagenesis. Furthermore, the variations may be synthetically produced sequences. The allelic variants may be naturally occurring variants or synthetically produced variants or variants produced by recombinant DNA techniques.
The proteins encoded by the different variants of the nucleic acid molecules of the invention possess certain characteristics they have in common. These include for instance biological activity, molecular weight, immunological reactivity, conformation, etc., and physical properties, such as for instance the migration behavior in gel electrophoreses, chromatographic behavior, sedimentation coefficients, solubility, spectroscopic properties, stability, pH optimum, temperature optimum etc.
The biological activity of the AIP protein, in particular the capacity to interact with Ser/Thr kinase Akt can be tested as described, e.g., in the Examples. In particular, it can be tested by a yeast two-hybrid system as described in Example 1.
A common characteristic of the proteins encoded by the nucleic acid molecules according to the invention is preferably that they contain a FYVE domain. Furthermore, they preferably contain five WD-40 repeats.

The nucleic acid molecules of the invention can be DNA molecules, in particular genomic DNA or cDNA. Moreover, the nucleic acid molecules of the invention may be RNA molecules. The nucleic acid molecules of the invention can be obtained for instance from natural sources or may be produced synthetically or by recombinant techniques, such as PCR.

The nucleic acid molecules of the invention now allow host cells to be prepared which produce recombinant proteins having the activity of an AIP protein of high purity and/or in sufficient quantities, as well as genetically engineered cells possessing an increased or reduced activity of these proteins.

The invention also relates to oligonucleotides specifically hybridizing to a nucleic acid molecule of the invention. Such oligonucleotides have a length of preferably at least 10, in particular at least 15, and particularly preferably of at least 50 nucleotides. They are characterized in that they specifically hybridize to the nucleic acid molecules of the invention, that is to say that they do not or only to a very minor extent hybridize to nucleic acid sequences encoding other proteins. The oligonucleotides of the invention can be used for instance as primers for amplification techniques such as the PCR reaction or as a hybridization probe to isolate related genes.

Moreover, the invention relates to vectors, in particular plasmids, cosmids, viruses, bacteriophages and other vectors commonly used in genetic engineering, which contain the above-described nucleic acid molecules of the invention. In a preferred embodiment of the invention, the vectors of the invention are suitable for the transformation of fungal cells, cells of microorganisms or animal cells, in particular mammalian cells. Preferably, such vectors are suitable for the transformation of human cells. In a particularly preferred embodiment such vectors are suitable for gene therapy purposes.

In another preferred embodiment, the nucleic acid molecules contained in the vectors are connected to regulatory elements ensuring transcription and synthesis of a translatable RNA in prokaryotic or eukaryotic cells.
The expression of the nucleic acid molecules of the invention in prokaryotic or eukaryotic cells, for instance in *Escherichia coli,* is interesting because it permits a more precise characterization of the biological activities of the proteins encoded by these molecules. Moreover, it is possible to express these proteins in such prokaryotic or eukaryotic cells which are free from interfering proteins. In addition, it is possible to insert different mutations into the nucleic acid molecules by methods usual in molecular biology (see for instance Sambrook et al., 1989, Molecular Cloning, A Laboratory Manual, 2nd edition, Cold Spring Harbor Laboratory Press, Cold Spring Harbor, NY), leading to the synthesis of proteins possibly having modified biological properties. In this regard it is on the one hand possible to produce deletion mutants in which nucleic acid molecules are produced by progressive deletions from the 5' or 3' end of the coding DNA sequence, and said nucleic acid molecules lead to the synthesis of correspondingly shortened proteins.
On the other hand, the introduction of point mutations is also conceivable at positions at which a modification of the amino acid sequence for instance influences the biological activity or the regulation of the protein.

Moreover, mutants possessing a modified substrate or product specificity can be prepared. Furthermore, it is possible to prepare mutants having a modified activity-temperature-profile.
Furthermore, in the case of expression in human cells, the introduction of mutations into the nucleic acid molecules of the invention allows the gene expression rate and/or the activity of the proteins encoded by the nucleic acid molecules of the invention to be reduced or increased.
For genetic engineering in prokaryotic cells, the nucleic acid molecules of the invention or parts of these molecules can be introduced into plasmids which permit mutagenesis or sequence modification by recombination of DNA sequences. Standard methods (see Sambrook et al., 1989, Molecular Cloning: A laboratory manual, 2nd edition, Cold Spring Harbor Laboratory Press, NY, USA) allow base exchanges to be performed or natural or synthetic sequences to be added. DNA fragments can be connected to each other by applying adapters and linkers to the fragments. Moreover, engineering measures which provide suitable restriction sites or remove surplus DNA or restriction sites can be used. In those cases, in which insertions, deletions or substitutions are possible, in vitro mutagenesis, "primer repair", restriction or ligation can be used. In general, a sequence analysis, restriction analysis and other methods of biochemistry and molecular biology are carried out as analysis methods.

Another embodiment of the invention relates to host cells, in particular prokaryotic or eukaryotic cells transformed and/or genetically modified with an above-described nucleic acid molecule of the invention or with a vector of the invention, and to cells derived from such transformed cells and containing a nucleic acid molecule or vector of the invention. In a preferred embodiment the host cell is genetically modified in such a way that it contains a nucleic acid molecule stably integrated into the genome. More preferably the nucleic acid molecule can be expressed so as to lead to the production of a protein having the biological activity of an AIP protein.
An overview of different expression systems is for instance contained in Methods in Enzymology 153 (1987), 385-516, in Bitter et al. (Methods in Enzymology 153 (1987), 516-544) and in Sawers et al. (Applied Microbiology and Biotechnology 46 (1996), 1-9), Billman-Jacobe (Current Opinion in Biotechnology 7 (1996), 500-4), Hockney (Trends in Biotechnology 12 (1994), 456-463), Griffiths et al., (Methods in Molecular Biology 75 (1997), 427-440). An overview of yeast expression systems is for instance given by Hensing et al. (Antonie van Leuwenhoek 67 (1995), 261-279), Bussineau et al. (Developments in Biological Standardization 83 (1994), 13-19), Gellissen et al. (Antonie van Leuwenhoek 62 (1992), 79-93, Fleer (Current Opinion in Biotechnology 3 (1992), 486-496), Vedvick (Current Opinion in Biotechnology 2 (1991), 742-745) and Buckholz (Bio/Technology 9 (1991), 1067-1072).
Expression vectors have been widely described in the literature. As a rule, they contain not only a selection marker gene and a replication-origin ensuring replication in the host selected, but also a bacterial or viral promoter, and in most cases a termination signal for transcription. Between the promoter and the termination signal there is in general at least one restriction site or a polylinker which enables the insertion of a coding DNA sequence. The DNA sequence naturally controlling the transcription of the corresponding gene can be used as the promoter sequence, if it is active in the selected host organism. However, this sequence can also be exchanged for other promoter sequences. It is possible to use promoters ensuring constitutive expression of the gene and inducible promoters which permit a deliberate control of the expression of the gene. Bacterial and viral promoter sequences possessing these properties are described in detail in the literature. Regulatory sequences for the expression in microorganisms (for instance *E*. *coli*, *S. cerevisiae*) are sufficiently described in the literature. Promoters permitting a particularly high expression of a downstream sequence are for instance the T7 promoter (Studier et al., Methods in Enzymology 185 (1990), 60-89), lacUV5, trp, trp-lacUV5 (DeBoer et al., in Rodriguez and Chamberlin (Eds), Promoters, Structure and Function; Praeger, New York, (1982), 462-481; DeBoer et al., Proc. Natl. Acad. Sci. USA (1983), 21-25), lp1, rac (Boros et al., Gene 42 (1986), 97-100). Inducible promoters are preferably used for the synthesis of proteins. These promoters often lead to higher protein yields than do constitutive promoters. In order to obtain an optimum amount of protein, a two-stage process is often used. First, the host cells are cultured under optimum conditions up to a relatively high cell density. In the second step, transcription is induced depending on the type of promoter used. In this regard, a tac promoter is particularly suitable which can be induced by lactose or IPTG (=isopropyl-β-D-thiogalactopyranoside) (deBoer et al., Proc. Natl. Acad. Sci. USA 80 (1983), 21-25). Termination signals for transcription are also described in the literature.
The transformation of the host cell with a nucleic acid molecule or vector according to the invention can be carried out by standard methods, as for instance described in Sambrook et al., (Molecular Cloning: A Laboratory Manual, 2nd edition (1989) Cold Spring Harbor Press, New York; Methods in Yeast Genetics, A Laboratory Course Manual, Cold Spring Harbor Laboratory Press, 1990). The host cell is cultured in nutrient media meeting the requirements of the particular host cell used, in particular in respect of the pH value, temperature, salt concentration, aeration, antibiotics, vitamins, trace elements etc. The protein according to the present invention can be recovered and purified from recombinant cell cultures by methods including ammonium sulfate or ethanol precipitation, acid extraction, anion or cation exchange chromatography, phosphocellulose chromatography, hydrophobic interaction chromatography, affinity chromatography, hydroxylapatite chromatography and lectin chromatography. Protein refolding steps can be used, as necessary, in completing configuration of the protein. Finally, high performance liquid chromatography (HPLC) can be employed for final purification steps.

Moreover, the invention relates to a protein and biologically active fragments thereof, which is encoded by a nucleic acid molecule according to the invention and to methods for its preparation, wherein a host cell according to the invention is cultured under conditions permitting the synthesis of the protein, and the protein is subsequently isolated from the cultured cells and/or the culture medium. The present invention also relates to the protein obtainable by such a method. The protein of the present invention may, e.g., be a naturally purified product, or a product of chemical synthetic procedures, or produced by recombinant techniques from a prokaryotic or eukaroytic host (for example, by bacterial, yeast, higher plant, insect and mammalian cells in culture). Depending upon the host employed in a recombinant production procedure, the protein of the presen invention may be glycosylated or may be non-glycosylated. The protein of the invention may also include an initial methionine amino acid residue. The protein according to the invention may be further modified to contain additional chemical moieties not normally part of the protein. Those derivatized moieties may, e.g., improve the stability, solubility, the biological half life or absorption of the protein. The moieties may also reduce or eliminate any undesirable side effects of the protein and the like. An overview for these moieties can be found, e.g., in Remington's Pharmaceutical Sciences (18th ed., Mack Publishing Co., Easton, PA (1990)). Polyethylene glycol (PEG) is an example for such a chemical moiety which has been used for the preparation of therapeutic proteins. The attachment of PEG to proteins has been shown to protect them against proteolysis (Sada et al., J. Fermentation Bioengineering 71 (1991), 137-139). Various methods are available for the attachment of certain PEG moieties to proteins (for review see: Abuchowski et al., in "Enzymes as Drugs"; Holcerberg and Roberts, eds. (1981), 367-383). Generally, PEG molecules are connected to the protein via a reactive group found on the protein. Amino groups, e.g. on lysines or the amino terminus of the protein are convenient for this attachment among others.

Furthermore, the present invention also relates to an antibody specifically recognizing a protein according to the invention. The antibody can be monoclonal or polyclonal and can be prepared according to methods well known in the art. The term "antibody" also comprises fragments of an antibody which still retain the binding specificity.
The protein according to the invention, its fragments or other derivatives thereof, or cells expressing them can be used as an immunogen to produce antibodies thereto. The present invention in particular also includes chimeric, single chain, and humanized antibodies, as well as Fab fragments, or the product of an Fab expression library. Various procedures known in the art may be used for the production of such antibodies and fragments.
Antibodies directed against a protein according to the present invention can be obtained, e.g., by direct injection of the protein into an animal or by administering the protein to an animal, preferably a non-human animal. The antibody so obtained will then bind the protein itself. In this manner, even a sequence encoding only a fragment of the protein can be used to generate antibodies binding the whole native protein. Such antibodies can then, e.g., be used to isolate the protein from tissue expressing that polypeptide or to detect it in a probe. For the preparation of monoclonal antibodies, any technique which provides antibodies produced by continuous cell line cultures can be used. Examples for such techniques include the hybridoma technique (Köhler and Milstein, Nature 256 (1975), 495-497), the trioma technique, the human B-cell hybridoma technique (Kozbor et al., Immunology Today 4 (1983), 72) and the EBV-hybridoma technique to produce human monoclonal antibodies (Cole et al., Monoclonal Antibodies and Cancer Therapy, Alan R. Liss, Inc. (1985), 77-96). Techniques describing the production of single chain antibodies (e.g., U.S. Patent 4,946,778) can be adapted to produce single chain antibodies to immunogenic proteins according to the present invention. Furthermore, transgenic mice may be used to express humanized antibodies directed against immunogenic proteins of the present invention.

The present invention also relates to a pharmaceutical composition comprising a protein according to the invention or a nucleic acid molecule according to the invention which is suitable to express such a protein in target cells, and optionally a pharmaceutically acceptable carrier or excipient.

Moreover, the present invention also relates to the use of a protein according to the invention or of a nucleic acid molecule according to the invention which is suitable to express such a protein in target cells for the preparation of a pharmaceutical composition for the treatment of disorders selected from the group consisting of disorders associated with impaired vesicular transport, such as impaired endosomal transport, insulin resistance, non-insulin dependent diabetes mellitus (NIDDM), obesity, aging and cardiovascular diseases. Further examples of diseases which may be treated are diseases which result from an impaired insulin metabolism or from an insulin resistance, such as, e.g., atherosclerosis, hypertension, cellulitis, myocardial ischemia, stroke, polycystic ovarian syndrom, all forms of ovarian cancer, blindness, wound healing, burns and hypoglycemia. Furthermore, diseases can be treated which result from a reduced glucose tolerance, e.g., endocrinological disorders, such as Cushing's Syndrome, acromegaly, etc., liver insufficiencies, such as liver cirrhosis, etc., renal insufficiencies, such as glomerulonephritis, cystes, etc., neurological disorders associated with impaired muscle function, such as paraplegie, tetraplegie, poliomyelitis, etc., diseases caused by viral infections and disorders in general which are dependent on vesicular transport, e.g. neurological disorders dependent on synaptic transport, such as epilepsy, etc., disorders associated with impaired antibody production and/or secretion, such as plasmacytoma, etc., and autoimmune diseases.
Furthermore, the above-mentioned pharmaceutical composition may also be used for the activation or deactivation of the antibody production of B-cells.
AIP could be one of the postulated missing links between the insulin mediated Akt activation and the subsequent trigger of exocytosis of GLUT4 containing vesicles in order to stimulate glucose uptake in fat or muscle cells. Since normal GLUT4 vesicle translocation is believed to be abnormal in important metabolic disorders, e.g. insulin resistance, non-insulin dependent diabetes mellitus (NIDDM), or obesity, the present invention provides a possible therapeutical target to help to treat these disorders pharmacologically. The pathological complications, e.g., of diabetes are all based on hyperglycemia. Therefore successful treatment of diabetes depends on maintaining a tight blood glucose level either by diet or regular insulin injections. However, the optimal blood glucose concentration is very narrow. Thus, one of the most dangerous complications either after insulin administration or inappropriate caloric intake is a slight "overdose" of insulin, increased glucose uptake and a subsequent life-threatening hypoglycemia and insulin shock. Given that AIP positively regulates GLUT4 translocation and glucose uptake by recruiting activated Akt to GLUT4 containing endosomes, every small-molecule approach that interferes with either the binding of Akt to AIP, the recruitment of an Akt target via binding to AIP or with transmission of the exocytic signal would be extremely useful in counteracting, e.g., high insulin concentration.

The pharmaceutical compositions according to the invention may be suitable to be administered orally, rectally, parenterally, intracisternally, intradermally, intravaginally, intraperitoneally, topically (as by powders, ointments, gels, creams, drops or transdermal patch), bucally, or as an oral or nasal spray. By "pharmaceutically acceptable carrier or excipient" is meant a non-toxic solid, semisolid or liquid filter, diluent, encapsulating material or formulation auxiliary of any type. The term "parenteral" as used herein refers to modes of administration which include intravenous, intramuscular, intraperitoneal, intrasternal, subcutaneous and intraarticular injection and infusion. It is understood that, when administered to a human patient, the total daily usage of the pharmaceutical compositions of the present invention will be decided by the attending physician within the scope of sound medical judgement. The specific therapeutically effective dose level for any particular patient will naturally depend upon a variety of factors including the age, body weight, general health, sex and diet of the patient; the time of administration, route of administration, and rate of excretion of the composition; the duration of the treatment; drugs (such as a chemotherapeutic agent) used in combination or coincidental with the specific composition and the like. Suitable formulations can be found, e.g., in Remington's Pharmaceutical Sciences, Mack Publishing Company, Easton, PA.
The protein according to the present invention may also be employed by expression of such a protein in vivo, which is also referred to as "gene therapy".
Thus, for example, cells from a patient may be engineered with a polynucleotide (DNA or RNA) encoding a protein ex vivo, with the engineered cells then being provided to a patient to be treated with the protein. Such methods are well known in the art. For example, cells may be engineered by procedures known in the art by use of a retroviral particle containing RNA encoding a protein according to the present invention.

The present invention also relates to an antagonist of the protein according to the invention. Examples of antagonists are an antibody according to the invention or an antisense construct directed against a transcript of a nucleic acid molecule according to the invention or a nucleotide sequence encoding such an antisense construct.
The antisense approach is an approach well known in the art and it can be used to control gene expression through triple-helix formation or antisense DNA or RNA, both of which methods are based on binding of a polynucleotide to DNA or RNA. For example, the 5' coding portion of a nucleotide sequence which codes for the protein of the present invention is used to design an antisense RNA oligonucleotide of from about 10 to 40 base pairs in length. The antisense RNA oligonucleotide hybridizes to the corresponding DNA in vivo and blocks translation of the DNA molecule. The antisense oligonucleotides described above can be delivered directly into cells or corresponding nucleotide sequences may be delivered into the cells such that the antisense RNA or DNA may be expressed in vivo.

Another example for an antagonist of the protein according to the invention are fragments of the AIP protein which still bind to Akt and which do no longer lead to the normal biological response of the binding of AIP to Akt, e.g., the binding of the fragment prevents the insulin dependent uptake of glucose. Such fragments of the AIP protein can readily be determined by the person skilled in the art, e.g., by preparing corresponding deletion mutants and testing these mutants in an assay as described in Example 2 for binding to Akt and in an assay as described in Example 4 for their ability to prevent the insulin dependent uptake of glucose. A further example for an antagonist is a fragment of an Akt protein which binds to AIP and thereby prevents its interaction with Akt.
The present invention also relates to a pharmaceutical composition comprising an antagonist of the protein according to the invention and optionally a pharmaceutically acceptable carrier or excipient

The present invention furthermore relates to the use of an antagonist of the protein according to the invention for the preparation of a pharmaceutical composition for the treatment of the diseases mentioned already above.

Also described is a method for screening compounds to identify those which act as agonists of the AIP protein according to the invention. Such a method preferably comprises the steps of
(i) incubating cells of a highly insulin responsive cell line expressing a dominant-negative Akt protein with at least one compound to be tested;
(ii) measuring the glucose uptake of the cells;
(iii) selecting those compounds which lead to an increase in glucose uptake of the cells;
(iv) incubating the cells as defined in step (i) with the compound selected according to step (iii) in the presence of an inhibitor of FYVE domains;
(v) measuring the glucose uptake of the cells; and
(vi) selecting those compounds which do no longer induce glucose uptake in the presence of an inhibitor of FYVE domains.

This method is based on the reasoning that a compound which in step (i) induces glucose uptake despite the presence of a dominant-negative Akt protein must act downstream of Akt. However, if the glucose uptake is blocked by the presence of, e.g., TPEN which acts as an inhibitor of FYVE domains, the compound depends on an intact AIP protein and will therefore be an agonist for the Akt/AIP protein interaction. On the other hand, if the compound-induced glucose uptake is not abolished, then the compound acts downstream of AIP and is not a direct agonist of AIP.
An example for cells that can be used in the above-described method are differentiated mouse 3T3-L1 adipocytes. A dominant-negative Akt can be, e.g., a kinase-inactive allele of Akt. As a FYVE domain inhibitor in step (iv), e.g. Zn²⁺ chelators, such as TPEN, can be used. The determination of the glucose uptake in steps (ii) and (v) can be done as described, e.g., in Example 4.

Similarly, the present invention provides a method for screening compounds to identify those which act as antagonists of the AIP protein according to the invention. Such a method preferably comprises the steps of
(i) incubating an AIP protein with an Akt protein alone (as control) or with at least one compound to be tested;
(ii) determining whether the compound disrupts the interaction between Akt and AIP thereby identifying compounds that act as antagonists.

Such a method is preferably designed as an ELISA method wherein the AIP protein is, e.g., immobilized and the Akt protein is HA-tagged. The Akt protein that interacts with AIP can then be detected by incubation with anti-HA antibodies and subsequently with an, e.g., enzyme- or fluorescence-linked secondary antibody. An antagonist which disrupts the Akt/A1P interaction would lead to a decrease in the ELISA signal detected.

Furthermore, it is described a method for preparing a pharmaceutical composition comprising the steps of identifying an agonist or an antagonist of the AIP protein by one of the methods described above and formulating the identified agonist or antagonist in a pharmaceutical composition. For the formulation of the pharmaceutical composition the same applies as described above for the pharmaceutical compositions according to the invention.

Moreover, the present invention relates to a diagnostic kit or composition comprising the protein, the oligonucleotide, the nucleic molecule or the antibody according to the invention.
The oligonucleotide or the nucleic acid molecule according to the invention may, e.g., be used for determining whether an individual carries a mutation in the AIP gene, thereby allowing to determine whether the individual suffers from a disease or has the susceptibility for a disease related to the presence of mutations in the AIP gene.
A variety of techniques is available to detect mutations at the DNA level. For example, nucleic acids for diagnosis may be obtained from a patient's cells, such as from blood, urine, saliva, tissue biopsy and autopsy material. The genomic DNA may be used directly for detection or may be amplified enzymatically by using PCR prior to analysis. RNA or cDNA may also be used for the same purpose. As an example, PCR primers complementary to the nucleic acid encoding the AIP protein can be used to identify and analyze AIP mutations. Deletions and insertions can, e.g., be detected by a change in size of the amplified product in comparison to the normal genotype. Point mutations can be identified by hybridizing amplified DNA to radiolabeled AIP RNA or alternatively, radiolabeled AIP antisense DNA sequences. Perfectly matched sequences can be distinguished from mismatched duplexes by RNase A digestion or by differences in melting temperatures.
Genetic testing based on DNA sequence differences may be achieved by detection of alteration in electrophoretic mobility of DNA fragments in gels with or without denaturing agents. Small sequence deletions and insertions can be visualized by high resolution gel electrophoresis. DNA fragments of different sequences may be distinguished on denaturing formamide gradient gels in which the mobilities of different DNA fragments are retarded in the gel at different positions according to their specific melting or partial melting temperature.
The protein and/or the antibody according to the present invention can, e.g., be used in a diagnostic assay for detecting altered levels of a protein according to the invention in various tissues. An over- or under-expression of the protein compared to normal control tissue samples may detect the presence of a disease or susceptibility to a disease. Suitable assays are well known in the art and include radioimmunoassays, competitive-binding assays, Western Blot analysis, ELISA assays and "sandwich" assay.

These and other embodiments are disclosed and obvious to a skilled person and embraced by the description and the examples of the present invention. Additional literature regarding one of the above-mentioned methods, means and applications, which can be used within the meaning of the present invention, can be obtained from the state of the art, for instance from public libraries for instance by the use of electronic means. This purpose can be served inter alia by public databases, such as the "medline", which are accessible via internet, for instance under the address http://www.ncbi.nlm.nih.gov/PubMed/medline.html. Other databases and addresses are known to a skilled person and can be obtained from the internet, for instance under the address http://www.lycos.com. An overview of sources and information regarding patents and patent applications in biotechnology is contained in Berks, TIBTECH 12 (1994), 352-364.
All of the above cited disclosures of patents, publications and database entries are specifically incorporated herein by reference in their entirety to the same extent as if each such individual patent, publication or entry were specifically and individually indicated to be incorporated by reference.

The plasmid AIP referred to in the present application has been deposited at the internationally acknowledged deposit "Deutsche Sammlung von Mikroorganismen und Zellkulturen" (DSMZ), in Braunschweig, Germany, according to the requirements of the Budapest Treaty for international acknowlegdement of microorganism deposits for patenting.
Accession number: DSM13510
Date of deposit: May 24, 2000
- **Figure 1**: shows the nucleotide sequence and the deduced amino acid sequence of the AIP encoding cDNA insert in plasmid AIP (DSM13510)
- **Figure 2**: shows the results of experiments concerning the localization of the AIP protein (Example 2)
- **Figure 3**: shows the results of experiments concerning the biochemical interaction between AIP and Akt-1, i.e. a pull-down assay (Example 3)
- **Figure 4**: shows the results of experiments concerning the inactivation of the FYVE domain of the AIP protein (Example 4)
- **Figure 5**: shows a schematic overview of the mechanism of insulin-regulated-glucose uptake and of the presumable function of the AIP protein in this mechanism.

The following Examples further illustrate the invention.

### Example 1

### Isolation of a nucleotide sequence encoding an Akt interacting protein

Identification of interacting proteins by the yeast two-hybrid system is based upon the detection of the expression of a reporter gene, the transcription of which is dependent upon the reconstitution of a transcriptional regulator by the interaction of two proteins, each one fused to one half of the transcriptional regulator.
A given protein, called the bait, and a library of proteins, the prey, are expressed as fusion proteins to a DNA binding domain and a transcriptional regulatory domain, respectively.
In the present case, the DNA binding domain of the yeast transcriptional activator Gal4 is N-terminally fused to HA-tagged Akt-1 (plasmid pGBT9PheS-HA-Akt-1, CLONTECH) and a human B cell cDNA library (Durfee et al., Genes & Dev. 7 (1993), 555-569) is fused to the activation domain of Gal4 (plasmid pACT, CLONTECH).

Both plasmids are lithiumacetate-transformed (www.umanitoba homepage; in particular, www.umanitoba.ca/faculties/medicine/unitslbiochemlgietz/Trafo.html) into yeast strain Y153 that features a Gal4 dependent β-galactosidase and histidine expression (Durfee et al., (1993) loc. cit.). Transformants are plated and grown on minimal base medium (CLONTECH) lacking uracile (selection for Y153), tryptophane (selection for pGBT9PheS-HA-Akt-1), leucine (selection for pACT) and histidine (selection for induction of Ga14 transcriptional activity). The medium also contains 7.5mM 3-amino-triazol in order to repress basal Ga14 transcription. When the colony size reaches 2-3 mm, lift assays are performed to screen for β-galactosidase expression. For this purpose, yeast colonies are overlayed with duralon membranes and the attached cells are shock-freezed twice in liquid nitrogen in order to break up cells and liberate β-galactosidase. After a brief thawing period, the membranes are placed on Whatman 3MM paper soaked with a 5-bromo-4-chloro-3-indolyl-b-D-galactopyranosid (X-gal, 0.5mg/ml) containing buffer. Due to the conversion of X-gal, the β-galactosidase positive colonies appear blue after 2-12 hours of incubation at room temperature. The corresponding colonies are picked from the original plates, amplified and plasmid DNA is extracted using a BIO 101 Fast DNA kit (BIO101). The isolated DNA is transformed into competent E.coli DH5α cells using standard techniques. Bacterial cultures are streaked out onto LG agar plates containing chlorophenylalanine (2g/L). The pGBT9PheS-HA-Akt-1 transformed DH5α cells do not survive due to a mutated phenylalanine-tRNA-synthetase subunit, but only pACT harbouring cells are selected. Plasmids are conventionally isolated and are reintroduced together with pGBT9PheS-HA-Akt-1 into Y153 via lithiumacetate transformation. Growing colonies are screened again for β-galactosidase activity.
Of initially 16 clones, only two scored repeatedly positive after retransformation. One of them represents AIP. The nucleotide sequence of the insert of AIP was determined using standard methods.
The nucleotide and deduced amino acid sequence of AIP is shown in SEQ ID NOs: 1 and 2 and in Figure 1. In Figure 1 also the location of the FYVE domain and of the WD-40 repeats is shown. The 5 WD-40 repeats and the FYVE finger domain are boxed or highlighted, respectively.

The molecular weight of the encoded protein calculated on the basis of the amino acid sequence is 45.282,94 Da. When determined by SDS-PAGE it is about 44 kDa.

### Example 2

### Localisation of AlP depends on an intact FYVE finger

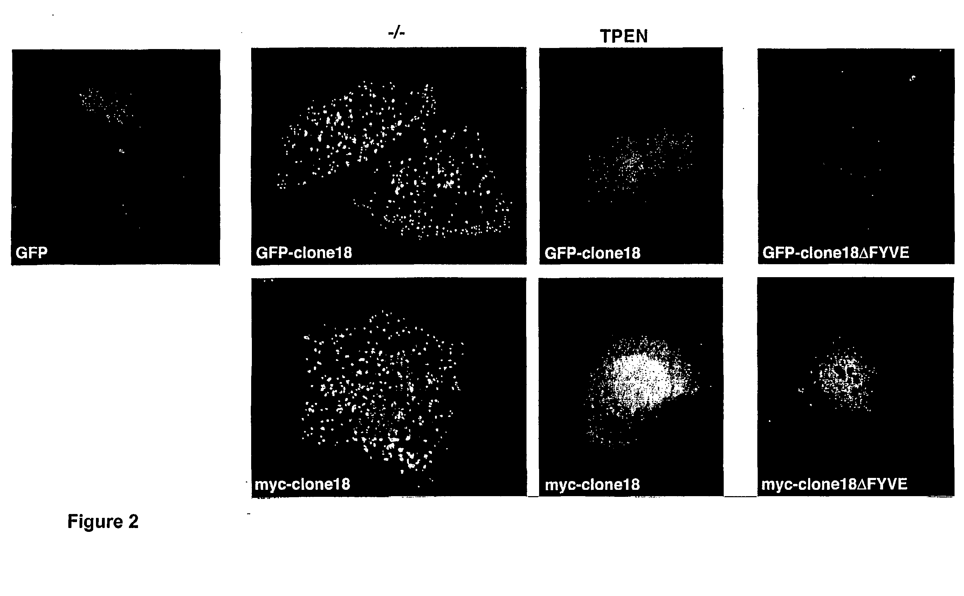

HeLa cells seeded on glass coverslips were transiently transfected with GFP, GFP-AIP (Figure 2; upper panels), myc-epitope-tagged AIP (Figure 2; lower part) coding plasmids or their respective FYVE-finger deletion mutants (Figure 2; rightmost upper and lower panel). 36 hours after transfection cells were fixed with 3% paraformaldehyde and in the case of GFP and GFP-AIP directly mounted onto glass slides. Myc-tagged AIP expressing cells were additionally permeabilized with 0.25% Triton X-100 for 5 min before they were incubated with a mouse anti-myc antibody and subsequently with a FITC-labelled anti-mouse antibody. After mounting, all samples were examined by confocal laser microscopy. Where indicated, transfected cells were incubated with 250 µm TPEN 30 min prior to fixation. The results of this experiment are shown in Figure 2. As can be seen, AIP is localised to early endosomal structures in human HeLa cells, that overexpress either an GFP-AIP fusion protein (upper panels) or myc-tagged AIP protein that can be specifically stained with an anti-myc antibody (lower panel). The pharmacological inactivation of either the AIP FYVE domain by incubating HeLa cells with Zn²⁺ chelators (TPEN) leads to complexation of zinc ions, disrupture of the FYVE domain structure causing the loss of the endosomal localisation and a concomitant diffuse cytoplasmic staining (panels indicated with TPEN). Accordingly, the deletion of the entire FYVE domain also leads to a cytoplasmic localisation of the overexpressed AIP. This localisation pattern does not depend on the relatively large GFP-fusion part of the protein, since an N-terminal myc-tag produces the same staining pattern when the cells are incubated with an anti-myc antibody and an appropriate secondary antibody to visualize myc-tagged AIP (rightmost upper and lower panel). The overexpression of GFP protein alone does not lead to any specific staining.

### Example 3

### Biochemical interaction between AIP and Akt-1

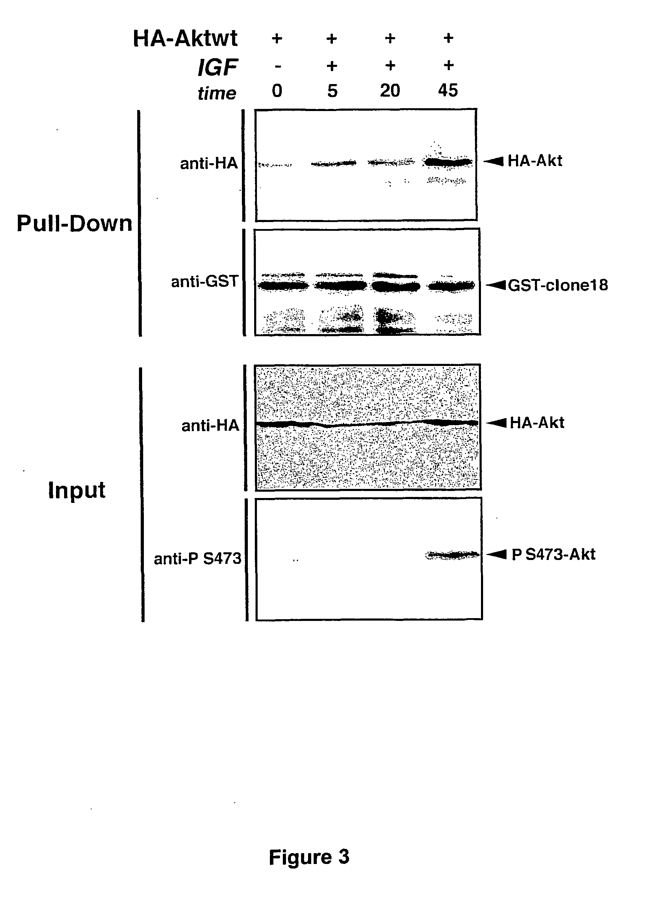

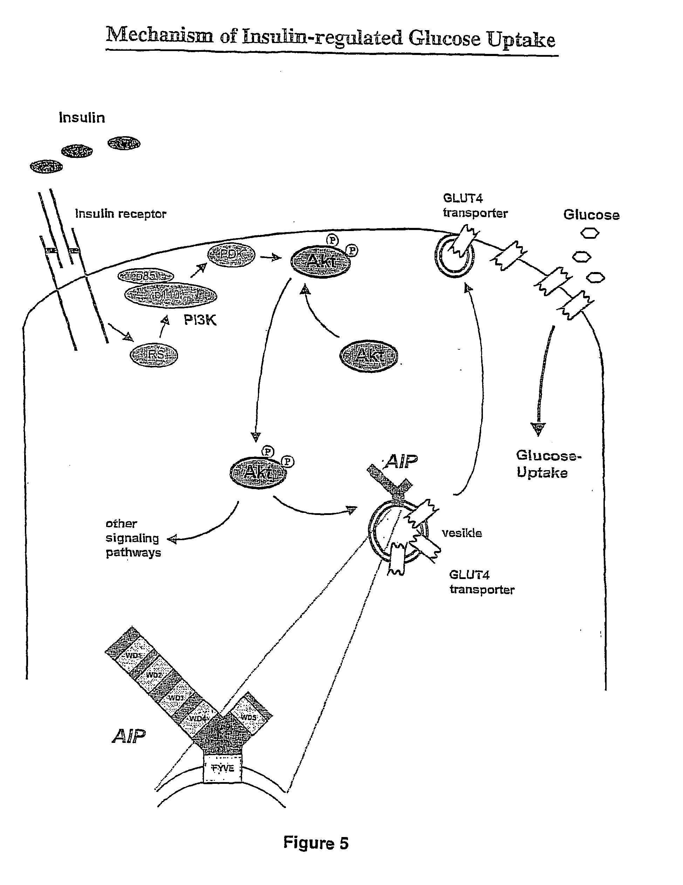

293T cells were transfected and allowed to express HA-tagged Akt-1 protein. After 20h of serum starvation cells were stimulated with insulin-like growth factor (IGF; 100ng/ml) for the indicated time periods (Figure 3; 0, 5, 20, 45 min). Afterwards, cells were washed and disrupted in mild lysis buffer containing 0.5% NP-40. Approximately 1% of the lysate was removed and boiled in SDS-sample buffer ("Input"). The remainder of the lysate was used in a so-called "pull-down assay". It was incubated for at least two hours with GSH-bead-immobilized recombinant GST-fused AIP purified from bacterial lysates. After several washes in lysis buffer, the beads were pelleted and the bound proteins were eluted in boiling SDS sample buffer, subjected to SDS-PAGE and western blotted with an anti-HA antibody ("pull-down", upper panel). The sample taken as "input" was also run on SDS-PAGE and western blotted with an anti-phospho-Ser473 Akt-1 antibody ("Input", upper panel) recognizing only activated Akt-1. After ECL detection and exposure to Hyperfilm, membranes were stripped off bound antibodies, re-blocked and incubated with an anti-GST antibody ("pull-down", lower panel) and an anti-HA antibody ("input", lower panel) in order to reveal comparable amounts of protein of GST-AIP and HA-Akt. The results of this experiment are shown in Figure 3. This shows that the interaction of Akt-1 and AIP is regulated and depends on the activation and phosphorylation state of Akt-1. When Akt-1 becomes activated after IGF stimulation it gets increasingly phosphorylated at Ser473 as shown with an Ser473 specific antibody lower panel input). The binding of Akt-1 to immobilised AIP is induced with very similar kinetics as the Ser473 phosphorylation. Therefore it is conceivable that after Akt-1 became activated and phosphorylated at the plasma membrane it diffuses through the cytoplasm where it binds to endosomally localised AIP due to ist phosphorylation at Ser473.

### Example 4

### Inactivation of FYVE finger proteins abolishes insulin stimulated glucose uptake

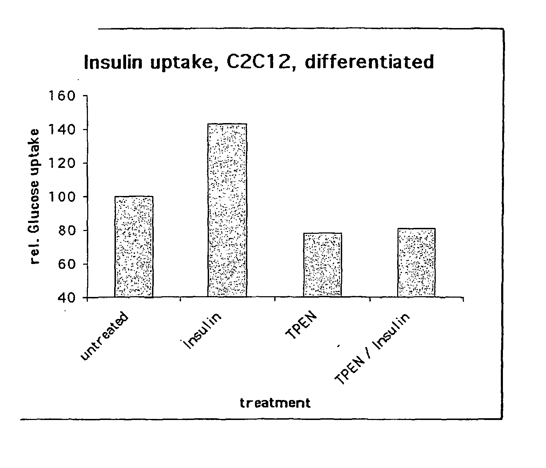

Mouse C2C12 fibroblasts were induced to differentiate and fuse into myotubes. 3 days after complete differentiation cells were starved for 6h and incubated with TPEN (250µm) for 30 min followed by incubation with insulin (200nM) for 2 h. TPEN is a Zn²⁺ chelator and inactivates the FYVE domain of AIP. Glucose uptake was determined by co-incubation with D-[¹⁴C]-glucose (100nCi) during insulin stimulation. After stimulation, the cells were washed, lysed in 0.05 M NaOH and glucose was quantitated by liquid scintillation counting. The results of this experiment are shown in Figure 4. As can be seen, the insulin-induced glucose uptake into the differentiated muscle cells can be fully reverted by preincubation with the FYVE domain inactivating compound TPEN. This shows that FYVE domain containing proteins are necessary for a regulated glucose uptake. Given the endosomal localisation of AIP and the already described involvement of Akt isoformes in glucose receptor translocation, it is very likely, that endosomal targeting of Akt isoformes, most probably mediated via AIP, is a positive regulator for GLUT4 tranlocation and glucose uptake.

## Claims

1. A nucleic acid molecule encoding an Akt interacting protein (AIP) selected from the group consisting of
(a) nucleic acid molecules encoding a protein which comprises the amino acid sequence indicated in SEQ ID NO: 2 or the amino acid sequence as encoded by the cDNA insert contained in plasmid DSM13510;
(b) nucleic acid molecules comprising the nucleotide sequence of the coding region indicated in SEO ID NO: 1 or the nucleotide sequence of the coding region of the cDNA insert contained in plasmid DSM13510;
(c) nucleic acid molecules encoding a protein, the amino acid sequence of which has a homology of at least 65% to the amino acid sequence indicated in SEQ ID NO: 2;
(d) nucleic acid molecules the complementary strand of which hybridizes to a nucleic acid molecule as defined in (a) or (b) and the sequence of which has a homology of at least 70% to the coding region of the sequence as shown in SEQ ID NO: 1; and
(e) nucleic acid molecules, the nucleotide sequence of which deviates because of the degeneracy of the genetic code from the sequence of the nucleic acid molecules as defined in any one of (a), (b), (c) or (d).

2. A vector containing the nucleic acid molecule of claim 1.

3. The vector of claim 2, wherein the nucleic acid molecule is linked to regulatory elements ensuring transcription in eukaryotic and prokaryotic cells.

4. A host cell, which is not a human embryonic or germ line all, which is genetically modified with a nucleic acid molecule of claim 1 or with a vector of claim 2 or 3.

5. A method for the production of an Akt interacting protein (AIP) encoded by a nucleic acid molecule of claim 1 in which the host cell of claim 4 is cultivated under conditions allowing for the expression of the protein and in which said AIP protein is isolated from the cells and/or the culture medium.

6. An AIP protein encoded by the nucleic acid molecule of claim 1 or obtainable by the method of claim 5.

7. An antibody specifically recognizing the protein of claim 6.

8. A pharmaceutical composition comprising the protein of claim 6 or a nucleic acid molecule of claim 1.

9. Use of the protein of claim 6 or of the nucleic acid molecule of claim 1 for the preparation of a pharmaceutical composition for the treatment of a disorder selected from the group consisting of disorders associated with impaired vesicular transport such as impaired endosomal transport, insulin resistance, non-insulin dependent diabetes mellitus (NIDDM), obesity, aging and cardiovascular diseases, diseases which result from an impaired insulin metabolism or from an insulin resistance, such as, e.g., atherosclerosis, hypertension, cellulitis, myocardial ischemia, stroke, polycystic ovarian syndrom, all forms of ovarian cancer, blindness, wound healing, burns and hypoglycemia, diseases which result from a reduced glucose tolerance, e.g., endocrinological disorders, such as Cushing's Syndrome, acromegaly, etc., liver insufficiencies, such as liver cirrhosis, etc., renal insufficiencies, such as glomerulonephritis, cystes, etc., neurological disorders associated with impaired muscle function, such as paraplegie, tetraplegie, poliomyelitis, etc., and disorders in general which are dependent on vesicular transport, e.g. neurological disorders dependent on synaptic transport, such as epilepsy, etc.

10. An antagonist of the protein of claim 6 which is
(i) an antibody of claim 7; or
(ii) an antisense construct directed against a transcript of a nucleic acid molecule of claim 1; or
(iii) a fragment of the polypeptide of claim 6 which still binds to Akt and the binding of which to Akt prevents the insulin dependent uptake of glucose by cells.

11. A pharmaceutical composition comprising the antagonist of claim 10.

12. Use of the antagonist of claim 10 which disrupts the interaction between Akt and the protein of claim 6 thereby preventing the insulin dependent uptake of glucose by cells for the preparation of a pharmaceutical composition for the treatment of a disorder selected from the group consisting of disorders associated with impaired vesicular transport such as diseases caused by viral infections and disorders in general which are dependent on vesicular transport, e.g. neurological disorders dependent on synaptic transport, such as epilepsy, etc., disorders associated with impaired antibody production and/or secretion, such as plasmacytoma, etc., and autoimmune diseases, or for the activation or deactivation of the antibody production of B-cells.

13. A diagnostic composition comprising the nucleic acid molecule of claim 1, the protein of claim 6 and/or the antibody of claim 7.

14. A method for screening compounds to identify those which act as antagonists of the protein of claim 6, comprising the steps of
(i) incubating a protein of claim 6 with an Akt protein alone (as control) or with at least one compound to be tested;
(ii) determining whether the compound disrupts the interaction between Akt and AIP thereby identifying compounds that act as antagonists.

## Patentansprüche

1. Nucleinsäuremolekül, welches ein Akt-interagierendes Protein (AIP) kodiert, ausgewählt aus der Gruppe, bestehend aus:
(a) Nucleinsäuremolekülen, die ein Protein kodieren, das die in SEQ ID NO: 2 angegebene Aminosäuresequenz oder die wie durch das im Plasmid DSM13510 enthaltene cDNA-Insert kodierte Aminosäuresequenz umfasst;
(b) Nucleinsäuremolekülen, welche die Nucleotidsequenz der in SEQ ID NO: 1 angegebenen kodierenden Region oder die Nucleotidsequenz der kodierenden Region des im Plasmid DSM13510 enthaltenen cDNA-Inserts umfassen;
(c) Nucleinsäuremolekülen, die ein Protein kodieren, dessen Aminosäuresequenz eine Homologie von mindestens 65% mit der in SEQ ID NO: 2 angegebenen Aminosäuresequenz aufweist;
(d) Nucleinsäuremolekülen, deren komplementärer Strang an ein wie in (a) oder (b) definiertes Nucleinsäuremolekül hybridisiert und deren Sequenz eine Homologie von mindestens 70% mit der kodierenden Region der wie in SEQ ID NO: gezeigten Sequenz aufweist, und
(e) Nucleinsäuremolekülen, deren Nucleotidsequenz wegen der Degeneriertheit des genetischen Codes von der Sequenz der wie in einem von (a), (b), (c) oder (d) definierten Nucleinsäuremoleküle abweicht.

2. Vektor, der das Nucleinsäuremolekül nach Anspruch 1 enthält.

3. Vektor nach Anspruch 2, wobei das Nucleinsäuremolekül mit regulatorischen Elementen verbunden ist, die eine Transkription in eukaryotischen und prokaryotischen Zellen sicherstellen.

4. Wirtszelle, bei der es sich nicht um eine menschliche embryonale oder Keimbahnzelle handelt, die mit einem Nucleinsäuremolekül nach Anspruch 1 oder mit einem Vektor nach Anspruch 2 oder 3 genetisch modifiziert ist.

5. Verfahren zur Herstellung eines Akt-interagierenden Proteins (AIP), das durch ein Nucleinsäuremolekül nach Anspruch 1 kodiert wird, wobei die Wirtszelle nach Anspruch 4 unter Bedingungen gezüchtet wird, Welche die Expression des Proteins ermöglichen, und wobei das AIP-Protein aus den Zellen und/oder dem Kulturmedium isoliert wird.

6. AIP-Protein, das durch das Nucleinsäuremolekül nach Anspruch 1 kodiert wird oder durch das Verfahren nach Anspruch 5 erhalten werden kann.

7. Antikörper, der das Protein nach Anspruch 6 spezifisch erkennt.

8. Pharmazeutische Zusammensetzung, umfassend das Protein nach Anspruch 6 oder ein Nucleinsäuremolekül nach Anspruch 1.

9. Verwendung des Proteins nach Anspruch 6 oder des Nucleinsäuremoleküls nach Anspruch 1 zur Herstellung einer pharmazeutischen Zusammensetzung zur Behandlung einer Störung, ausgewählt aus der Gruppe, bestehend aus Störungen, die mit einem beeinträchtigten vesikulären Transport assoziiert sind, wie zum Beispiel beeinträchtigter endosomaler Transport, Insulinresistenz, nicht insulinabhängiger Diabetes mellitus (NIDDM), Fettsucht, Altern und kardiovaskuläre Erkrankungen, Erkrankungen, die sich aus einem beeinträchtigten Insulinmetabolismus oder aus einer Insulinresistenz ergeben, wie zum Beispiel Atherosklerose, Hypertonie, Cellulitis, Myokardischämie, Schlag-anfall; Syndrom der polyzystischen Ovarien, alle Formen von Ovarialkrebs, Blindheit, Wundheilung, Verbrennungen und Hypoglykämie, Erkrankungen, die sich aus einer reduzierten Glucosetoleranz ergeben, z. B. endokrinologische Störungen, wie zum Beispiel Cushing-Syndrom, Akromegalie usw., Leberinsuffizienzen, wie zum Beispiel Leberzirrhose usw., Niereninsuffizienzen, wie zum Beispiel Glomerulonephritis, Cysten usw., neurologischen Störungen, die mit einer beeinträchtigten Muskelfunktion assoziiert sind, wie zum Beispiel Paraplegie, Tetraplegie, Poliomyelitis usw., und Störungen im Allgemeinen, die von vesikulärem Transport abhängen, z. B. neurologische Störungen, die von synaptischem Transport abhängen, wie zum Beispiel Epilepsie usw.

10. Antagonist des Proteins nach Anspruch 6, bei dem es sich um
(i) einen Antikörper nach Anspruch 7 oder
(ii) ein gegen ein Transkript eines Nucleinsäuremoleküls nach Anspruch 1 gerichtetes Antisense-Konstrukt oder
(iii) ein Fragment des Polypeptids nach Anspruch 6, das noch an Akt bindet und dessen Bindung an Akt die insulinabhängige Aufnahme von Glucose durch Zellen verhindert,
handelt.

11. Pharmazeutische Zusammensetzung, umfassend den Antagonisten nach Anspruch 10.

12. Verwendung des Antagonisten nach Anspruch 10, der die Wechselwirkung zwischen Akt und dem Protein nach Anspruch 6 unterbricht und **dadurch** die insulinabhängige Aufnahme von Glucose durch Zellen verhindert, zur Herstellung einer pharmazeutischen Zusammensetzung zur Behandlung einer Störung, ausgewählt aus der Gruppe, bestehend aus Störungen, die mit einem beeinträchtigten vesikulären Transport assoziiert sind, wie zum Beispiel durch Virusinfektionen verursachte Erkrankungen und Störungen im Allgemeinen, die von vesikulärem Transport abhängen, z. B. neurologische Störungen, die von synaptischem Transport abhängen, wie zum Beispiel Epilepsie usw., Störungen, die mit einer beeinträchtigten Antikörperproduktion und/oder -sekretion assoziiert sind, wie zum Beispiel ein Plasmacytom usw., und Autoimmunerkrankungen, oder zur Aktivierung oder Deaktivierung der Antikörperproduktion von B-Zellen.

13. Diagnostische Zusammensetzung, umfassend das Nucleinsäuremolekül nach Anspruch 1, das Protein nach Anspruch 6 und/oder den Antikörper nach Anspruch 7.

14. Verfahren zum Durchmustern von Verbindungen, um diejenigen zu identifizieren, die als Antagonisten des Proteins nach Anspruch 6 wirken, umfassend die Schritte
(i) Inkubieren eines Proteins nach Anspruch 6 mit einem Akt Protein allein (als Kontrolle) oder mit mindestens einer zu testenden Verbindung;
(ii) Bestimmen, ob die Verbindung die Wechselwirkung zwischen Akt und AIP unterbricht, wodurch Verbindungen identifiziert werden, die als Antagonisten wirken.

## Revendications

1. Molécule d'acide nucléique codant pour une protéine interagissant avec l'Akt (AIP) choisie dans le groupe constitué par :
(a) des molécules d'acide nucléique codant pour une protéine qui comprend la séquence d'acides aminés indiquée dans SEQ ID NO : 2 ou la séquence d'acides aminés telle qu'elle est codée par le segment d'insertion d'ADNc contenu dans le plasmide DSM13510 ;
(b) des molécules d'acide nucléique comprenant la séquence de nucléotides de la région codante indiquée dans SEQ ID NO : 1 ou la séquence de nucléotides de la région codante du segment d'insertion d'ADNc contenu dans le plasmide DSM13510 ;
(c) des molécules d'acide nucléique codant pour une protéine dont la séquence d'acides aminés présente une homologie d'au moins 65 % par rapport à la séquence d'acides aminés indiquée dans SEQ ID NO : 2 ;
(d) des molécules diacide nucléique dont le brin complémentaire s'hybride à une molécule d'acide nucléique telle que définie en (a) ou (b) et dont la séquence présente une homologie d'au moins 70 % par rapport à la région codante de la séquence telle que présentée dans SEQ ID NO : 1 ; et
(e) des molécules d'acide nucléique dont la séquence de nucléotides dévie, en raison de la dégénérescence du code génétique, de la séquence des molécules d'acide nucléique telles que définies en (a), (b), (c) ou (d).

2. Vecteur contenant la molécule d'acide nucléique de la revendication 1.

3. Vecteur de la revendication 2, dans lequel la molécule d'acide nucléique est liée à des éléments régulateurs assurant la transcription dans des cellules eucaryotes et procaryotes.

4. Cellule hôte, qui n'est pas une cellule embryonnaire humaine ou germinale, qui est génétiquement modifiée avec une molécule d'acide nucléique de la revendication 1 ou avec un vecteur de la revendication 2 ou 3.

5. Procédé pour produire une protéine interagissant avec l'Akt (AIP) codée par une molécule d'acide nucléique de la revendication 1, dans lequel la cellule hôte de la revendication 4 est cultivée dans des conditions permettant l'expression de la protéine et dans lequel ladite protéine AIP est isolée des cellules et/ou du milieu de culture.

6. Protéine AIP codée par la molécule d'acide nucléique selon la revendication 1 ou pouvant être obtenue par le procédé de la revendication 5.

7. Anticorps reconnaissant spécifiquement la protéine de la revendication 6.

8. Composition pharmaceutique comprenant la protéine de la revendication 6 ou une molécule d'acide nucléique de la revendication 1.

9. Utilisation de la protéine de la revendication 6 ou de la molécule d'acide nucléique de la revendication 1 pour la préparation d'une composition pharmaceutique pour le traitement d'un trouble choisi dans le groupe constitué par les troubles associés au transport vésiculaire pertubé, tel que le transport endosomal pertubé, la résistance à l'insuline, le diabète sucré non-insulinodépendant (NIDDM), l'obésité, le vieillissement et les maladies cardiovasculaires, les maladies qui résultent d'un métabolisme pertubé de l'insuline ou d'une résistance à l'insuline, telles que, par exemple l'athérosclérose, l'hypertension, la cellulite, l'ischémie myocardique, l'accident vasculaire celebral, le syndrome des ovaires polykystiques, toutes les formes de cancer de l'ovaire, la cécité, la cicatrisation des plaies, les brûlures et l'hypoglycémie, les maladies qui résultent d'une tolérance réduite au glucose, par exemple les troubles endocriniens tels que le syndrome de Cushing, l'acromégalie, *etc*., les insuffisances hépatiques telles que la cirrhose du foie, *etc*., les insuffisances rénales telles que la glomérulonéphrite, les kystes, *etc.*, les troubles neurologiques associés à une fonction musculaire pertubée telle que la paraplégie, la tétraplégie, la polyomyélite, etc. et les troubles en général qui dépendent du transport vésiculaire, par exemple les troubles neurologiques dépendant du transport synaptique tels que l'épilepsie, etc.

10. Antagoniste de la protéine de la revendication 6, qui est :
(i) un anticorps de la revendication 7 ; ou
(ii) un construit d'antisens dirigé contre un produit de transcription d'une molécule d'acide nucléique de la revendication 1 ; ou
(iii) un fragment du polypeptide de la revendication 6, qui se lie encore à l'Akt et dont la liaison à l'Akt empêche l'absorption insulinodépendante du glucose par les cellules.

11. Composition pharmaceutique comprenant l'antagoniste de la revendication 10.

12. Utilisation de l'antagoniste de la revendication 10 qui interrompt l'interaction entre l'Akt et la protéine de la revendication 6, en empêchant ainsi l'absorption insulinodépendante du glucose par les cellules, pour la préparation d'une composition pharmaceutique pour le traitement d'un trouble choisi dans le groupe constitué par les troubles associés au transport vésiculaire pertubé tels que les maladies causées par des infections virales et les troubles en général qui dépendent du transport vésiculaire, par exemple les troubles neurologiques dépendant du transport synaptique tels que l'épilepsie, etc., les troubles associés à la production et/ou sécrétion anormale d'anticorps tels que le plasmacytome, *etc.* et les maladies autoimmunes ou pour l'activation ou la désactivation de la production d'anticorps des cellules B.

13. Composition pour le diagnostic, comprenant la molécule d'acide nucléique de la revendication 1, la protéine de la revendication 6 et/ou l'anticorps de la revendication 7.

14. Procédé pour cribler des composés afin d'identifier ceux qui agissent en tant qu'antagonistes de la protéine de la revendication 6, comprenant les étapes consistant à :
(i) incuber une protéine de la revendication 6 avec une protéine Akt seule (témoin) ou avec au moins un composé à tester ;
(ii) déterminer si le composé interrompt l'interaction entre l'Akt et l'AIP, en identifiant ainsi les composés qui agissent comme antagonistes.


### SEQUENCE LISTING

<110> Mölling, Karin
<120> Nucleic acid molecules encoding a protein interacting with Ser/Thr kinase Akt-1
<130> E 1630 PCT
<140>
   <141>
<160> 2
<170> PatentIn Ver. 2.1
<210> 1
   <211> 2098
   <212> DNA
   <213> Homo sapiens
<220>
   <221> CDS
   <222> (208)..(1410)
<400> 1
   ttattcgatg atgaagatac cccaccaaac ccaaaaaaag agatctggaa ttcggatcct 60
   cgaggccacg aaggcccgcc ggtttccggc gttccgctcc ggccagccag agtctctgtc 120
   tcaacctgtg tccgtgctcc agcagtctcc tcagcccggc cccgcggcgc ggttggcggc 180
   ggcgccccag gcgcgccccc tcctccg atg gcg gcg gag atc cag ccc aag cct 234
                                 Met Ala Ala Glu Ile Gln Pro Lys Pro
                                  1               5
   ctg acc cgc aag ccg atc ctg ctg cag cgg atg gag ggg tcc cag gag   282
   Leu Thr Arg Lys Pro Ile Leu Leu Gln Arg Met Glu Gly Ser Gln Glu
    10                 15                  20                  25
   gtg gtg aat atg gcc gtg atc gtg ccc aaa gag gag ggc gtc atc agc   330
   Val Val Asn Met Ala Val Ile Val Pro Lys Glu Glu Gly Val Ile Ser
                   30                  35                  40
   gtc tcc gag gac agg aca gtt cgt gtt tgg tta aag aga gac agt gga   378
   Val Ser Glu Asp Arg Thr Val Arg Val Trp Leu Lys Arg Asp Ser Gly
               45                  50                  55
   cag tat tgg cca agc gta tac cat gca atg cct tct cca tgt tca tgc   426
   Gln Tyr Trp Pro Ser Val Tyr His Ala Met Pro Ser Pro Cys Ser Cys
           60                  65                  70
   atg tct ttt aac ccg gaa aca aga aga ctg tcc ata ggt cta gac aat   474
   Met Ser Phe Asn Pro Glu Thr Arg Arg Leu Ser Ile Gly Leu Asp Asn
       75                  80                  85
   ggt aca atc tca gag ttt ata ttg tca gaa gat tat aac aag atg act   522
   Gly Thr Ile Ser Glu Phe Ile Leu Ser Glu Asp Tyr Asn Lys Met Thr
    90                 95                  100                 105
   cct gtg aaa aac tat caa gcg cat cag agc aga gtg acg atg atc ctg    570
   Pro Val Lys Asn Tyr Gln Ala His Gln Ser Arg Val Thr Met Ile Leu
                   110                 115                 120
   ttt gtc ctg gag ctg gag tgg gtg ctg agc aca gga cag gac aag caa    618
   Phe Val Leu Glu Leu Glu Trp Val Leu Ser Thr Gly Gln Asp Lys Gln
               125                 130                 135
   ttt gcc tgg cac tgc tct gag agt ggg cag cgc ctg gga ggt tat cgg    666
   Phe Ala Trp His Cys Ser Glu Ser Gly Gln Arg Leu Gly Gly Tyr Arg
           140                 145                 150
   acc agt gct gtg gcc tca ggc ctg caa ttt gat gtt gaa acc cgg cat    714
   Thr Ser Ala Val Ala Ser Gly Leu Gln Phe Asp Val Glu Thr Arg His
       155                 160                 165
   gtg ttt atc ggt gac cac tca ggc caa gta aca atc ctc aaa ctg gag    762
   Val Phe Ile Gly Asp His Ser Gly Gln Val Thr Ile Leu Lys Leu Glu
   170                 175                 180                 185
   caa gaa aac tgc acc ctg gtc aca aca ttc aga gga cac aca ggt ggg    810
   Gln Glu Asn Cys Thr Leu Val Thr Thr Phe Arg Gly His Thr Gly Gly
                   190                 195                 200
   gtg acc gct ctc tgt tgg gac cca gtc cag cgg gtg ttg ttc tca ggc    858
   Val Thr Ala Leu Cys Trp Asp Pro Val Gln Arg Val Leu Phe Ser Gly
               205                 210                 215
   agt tca gat cac tct gtc atc atg tgg gac atc ggt ggg aga aaa gga    906
   Ser Ser Asp His Ser Val Ile Met Trp Asp Ile Gly Gly Arg Lys Gly
           220                 225                 230
   aca gcc atc gag ctc caa gga cac aac gac aga gtc cag gcc ctc tcc    954
   Thr Ala Ile Glu Leu Gln Gly His Asn Asp Arg Val Gln Ala Leu Ser
       235                 240                 245
   tat gca cag cac acg cga caa ttg atc tcc tgt ggc ggt gat ggt ggg    1002
   Tyr Ala Gln His Thr Arg Gln Leu Ile Ser Cys Gly Gly Asp Gly Gly
   250                 255                 260                 265
   att gtc gtc tgg aac atg gac gtg gag agg cag gag acc cct gaa tgg    1050
   Ile Val Val Trp Asn Met Asp Val Glu Arg Gln Glu Thr Pro Glu Trp
                   270                 275                 280
   ttg gac agt gat tcc tgc caa aag tgt gat cag cct ttc ttc tgg aac    1098
   Leu Asp Ser Asp Ser Cys Gln Lys Cys Asp Gln Pro Phe Phe Trp Asn
               285                 290                 295
   ttc aag caa atg tgg gac agt aag aaa att ggt cta aga cag cac cac    1146
   Phe Lys Gln Met Trp Asp Ser Lys Lys Ile Gly Leu Arg Gln His His
           300                 305                 310
   tgc cgc aag tgt ggg aag gcc gtc tgt ggc aag tgc agc tcc aag cgc    1194
   Cys Arg Lys Cys Gly Lys Ala Val Cys Gly Lys Cys Ser Ser Lys Arg
       315                 320                 325
   tcc tcc atc ccc ctg atg ggc ttc gag ttt gaa gtg agg gtc tgt gac    1242
   Ser Ser Ile Pro Leu Met Gly Phe Glu Phe Glu Val Arg Val Cys Asp
   330                 335                 340                 345
   agc tgc cac gag gcc atc aca gat gaa gaa cgt gca ccc aca gcc acc   1290
   Ser Cys His Glu Ala Ile Thr Asp Glu Glu Arg Ala Pro Thr Ala Thr
                   350                 355                 360
   ttc cat gac agt aaa cat aac att gtg cat gtg cat ttc gat gca acc   1338
   Phe His Asp Ser Lys His Asn Ile Val His Val His Phe Asp Ala Thr
               365                 370                 375
   aga gga tgg tta ctg act tct gga act gac aag gtt att aag ttg tgg   1386
   Arg Gly Trp Leu Leu Thr Ser Gly Thr Asp Lys Val Ile Lys Leu Trp
           380                 385                 390
   gat atg acc cca gtc gtg tct tga tgactctccc aggaatcaga aagatagtat  1440
   Asp Met Thr Pro Val Val Ser
       395                 400
   ttactaaaga aacggttgtt ttaacccaaa tcattaccag agtggtaaag cagacatgtg 1500
   agaagtaaga aagaaactaa agaccctgaa tgaatttgca gattacccat gtgcacagtg 1560
   gggacctggc cagtgagcac tcgcaagggg actcttccaa cttgttcata caatataaaa 1620
   gaagctattt ttttaacaaa tggtttatac agtctggctg tgctgcattg ttttgagtgt 1680
   accgaaaaat ctgtgtgggg tgtttaattt ttatactttt caacacccca ttttacttgt 1740
   tgctttgtca gagaaataag ggaggtatct actcagagta ttttggtcat tatactttct 1800
   gtgtttactt caacatgtgt cacgtggcca gcggcttttt cttctcttcc ctctgcacct 1860
   acctgcacct tctctgcctt tcctggaggg gatgtattta tgttatttat tcccagtgtt 1920
   tctgctttca tgtcctcctc agtggagaga tttggaaact catcatgtgg attcaccagc 1980
   cagctgctgg aattgcctga agagcgattt gtttgtaatg tctgcctcat tcacgttctt 2040
   atgaagtaga ggccttcgtg gcctcgagag atccactagt tctagagcgg ccgccacc   2098
<210> 2
   <211> 400
   <212> PRT
   <213> Homo sapiens
<400> 2
   Met Ala Ala Glu Ile Gln Pro Lys Pro Leu Thr Arg Lys Pro Ile Leu
    1               5                   10                  15
   Leu Gln Arg Met Glu Gly Ser Gln Glu Val Val Asn Met Ala Val Ile
               20                  25                  30
   Val Pro Lys Glu Glu Gly Val Ile Ser Val Ser Glu Asp Arg Thr Val
           35                  40                  45
   Arg Val Trp Leu Lys Arg Asp Ser Gly Gln Tyr Trp Pro Ser Val Tyr
       50                  55                  60
   His Ala Met Pro Ser Pro Cys Ser Cys Met Ser Phe Asn Pro Glu Thr
   65                  70                  75                  80
   Arg Arg Leu Ser Ile Gly Leu Asp Asn Gly Thr Ile Ser Glu Phe Ile
                   85                  90                  95
   Leu Ser Glu Asp Tyr Asn Lys Met Thr Pro Val Lys Asn Tyr Gln Ala
               100                 105                 110
   His Gln Ser Arg Val Thr Met Ile Leu Phe Val Leu Glu Leu Glu Trp
           115                 120                 125
   Val Leu Ser Thr Gly Gln Asp Lys Gln Phe Ala Trp His Cys Ser Glu
       130                 135                 140
   Ser Gly Gln Arg Leu Gly Gly Tyr Arg Thr Ser Ala Val Ala Ser Gly
   145                 150                 155                 160
   Leu Gln Phe Asp Val Glu Thr Arg His Val Phe Ile Gly Asp His Ser
                   165                 170                 175
   Gly Gln Val Thr Ile Leu Lys Leu Glu Gln Glu Asn Cys Thr Leu Val
               180                 185                 190
   Thr Thr Phe Arg Gly His Thr Gly Gly Val Thr Ala Leu Cys Trp Asp
           195                 200                 205
   Pro Val Gln Arg Val Leu Phe Ser Gly Ser Ser Asp His Ser Val Ile
       210                 215                 220
   Met Trp Asp Ile Gly Gly Arg Lys Gly Thr Ala Ile Glu Leu Gln Gly
   225                 230                 235                 240
   His Asn Asp Arg Val Gln Ala Leu Ser Tyr Ala Gln His Thr Arg Gln
                   245                 250                 255
   Leu Ile Ser Cys Gly Gly Asp Gly Gly Ile Val Val Trp Asn Met Asp
               260                 265                 270
   Val Glu Arg Gln Glu Thr Pro Glu Trp Leu Asp Ser Asp Ser Cys Gln
           275                 280                 285
   Lys Cys Asp Gln Pro Phe Phe Trp Asn Phe Lys Gln Met Trp Asp Ser
       290                 295                 300
   Lys Lys Ile Gly Leu Arg Gln His His Cys Arg Lys Cys Gly Lys Ala
   305                 310                 315                 320
   Val Cys Gly Lys Cys Ser Ser Lys Arg Ser Ser Ile Pro Leu Met Gly
                   325                 330                 335
   Phe Glu Phe Glu Val Arg Val Cys Asp Ser Cys His Glu Ala Ile Thr
               340                 345                 350
   Asp Glu Glu Arg Ala Pro Thr Ala Thr Phe His Asp Ser Lys His Asn
           355                 360                 365
   Ile Val His Val His Phe Asp Ala Thr Arg Gly Trp Leu Leu Thr Ser
       370                 375                 380
   Gly Thr Asp Lys Val Ile Lys Leu Trp Asp Met Thr Pro Val Val Ser
   385                 390                 395                 400